# EUROPEAN PATENT APPLICATION

(11) **EP 4 628 028 A1**
(43) Date of publication of application: **08.10.2025**
(21) Application number: 24909242.0
(22) Date of filing: 30.12.2024
(51) Int. Cl.: A61B 17/00

(54) **OCCLUDER, OCCLUDER MANUFACTURING APPARATUS, AND OCCLUDER SYSTEM**

(30) Priority: 23.02.2024 CN 202410204363; 04.07.2024 CN 202410889799
(71) Applicant: WYTD Medical Technology (Shenzhen) Co., Ltd., Shenzhen, Guangdong 518122 (CN)
(72) Inventor: KANG, Libiao, Shenzhen, Guangdong 518122 (CN); LIAO, Haiyan, Shenzhen, Guangdong 518122 (CN); CHEN, Chaosong, Shenzhen, Guangdong 518122 (CN); WANG, Chenwei, Shenzhen, Guangdong 518122 (CN)
(74) Representative: Dilg, Haeusler, Schindelmann Patentanwaltsgesellschaft mbH
(86) International application number: PCT/CN2024/144014
(87) International publication number: WO 2025/175929

(57) **Abstract**

The present invention provides an occluder, an occluder manufacturing device and an occluder system, wherein the occluder comprises a first occluding portion and a second occluding portion, and the first occluding portion and the second occluding portion are connected by a connecting portion; the first occluding portion and the second occluding portion are both composed of a mesh structure, the second occluding portion is provided with a lacing member, the lacing member is arranged in a surrounding manner and passes through meshes at an end of the second occluding portion in sequence, and a limiting member is arranged on the lacing member. The present invention reduces the inner diameter of the channel formed at the end of the second occluding portion by providing the lacing member and the limiting member, so as to improve the locking success rate of the occluder during surgery. In addition, the recyclable occluder system provided by the present invention can solve the problem that the occluder cannot be retracted after the umbrella body is formed.

## Description

### CROSS-REFERENCE TO RELATED DISCLOSURES

The present invention claims the priorities to Chinese patent applications with the filing No. 2024108897998 filed with the Chinese Patent Office on July 4, 2024, and entitled "OCCLUDER, OCCLUDER MANUFACTURING DEVICE AND OCCLUDER SYSTEM" and with the filing No. 2024102043630 filed with the Chinese Patent Office on February 23, 2024, and entitled "RECYCLABLE OCCLUDER SYSTEM", the entire contents of which are incorporated by reference into this disclosure.

### TECHNICAL FIELD

The present invention relates to the technical field of medical devices, and in particular to an occluder, an occluder manufacturing device and an occluder system.

### BACKGROUND ART

Patent foramen ovale is a congenital heart structural abnormality, which is caused by the failure of the heart channel that should close naturally after the baby is born to close. Currently, the main occluder used on the market is delivered to the patent foramen ovale defect through a catheter and released to block the patent foramen ovale defect to treat patent foramen ovale.

The existing occluder cooperates with a metal ring. After the occluder is delivered to the patent foramen ovale defect through a catheter and released, the medical staff delivers the metal ring to lock the occluder so that the occluder remains locked at the patent foramen ovale defect. At present, the locking effect between the occluder and the metal ring is not good.

### SUMMARY

In order to overcome the defect of poor locking effect between the existing occluder and the metal ring, the present invention provides an occluder, an occluder manufacturing device and an occluder system. The specific technical scheme is as follows.

The present invention provides an occluder, the occluder includes a first occluding portion and a second occluding portion, the first occluding portion and the second occluding portion are connected by a connecting portion; the first occluding portion and the second occluding portion are both composed of a mesh structure, the second occluding portion is equipped with a lacing member, the lacing member is arranged in a surrounding manner and passes through the mesh at the end of the second occluding portion in sequence, and a limiting member is arranged on the lacing member, the limiting member includes a plurality of limiting segments, and the plurality of limiting segments are connected on the lacing member and enclosed to form a closed pattern.

The occluder manufacturing device provided by the present invention is used to manufacture the above-mentioned occlude. The occluder manufacturing device comprises a columnar body, wherein a first end of the columnar body is provided with a plurality of first positioning members (A1₁, A1₂...A1ₙ) and a plurality of second positioning members (A2₁, A2₂...A2ₙ), and the second positioning member at the first end of the columnar body is located between two adjacent first positioning members of the first end of the columnar body, wherein a second end of the columnar body is also provided with a plurality of first positioning members (B1₁, B1₂...B1ₙ) and a plurality of second positioning members (B2₁, B2₂...B2ₙ); the second positioning member at the second end of the columnar body is located between two adjacent first positioning members of the second end of the columnar body.

The occluder manufacturing device is suitable for an occluder weaving method for manufacturing the occlude. The occluder weaving method comprises: fixing a single wire on A1₁, and passing the single wire through all the first positioning members and the second positioning members in the order of A1₁, B1₁, A2₁, B2₁...A1ₙ, B1ₙ, A2ₙ, B2ₙ to form a weaved body, wherein the single wire located between the first end of the columnar body and the second end of the columnar body is wound along the outer circumference of the columnar body, wherein, during the weaving process, the single wire is interwoven and passed through the weaved paths up and down to form a mesh structure; fixing a middle portion of the weaved body, and squeezing the two ends of the weaved body toward the middle portion of the weaved body to form an occluder having the first occluding portion and the second occluding portion.

An occluder system provided in the present invention comprises: the occluder and a conveying and cutting device;

a first auxiliary line, wherein one end of the first auxiliary line is arranged on an end surface of the first occluding portion of the occluder away from the second occluding portion of the occluder, the other end of the first auxiliary line passes through the second occluding portion through the limiting member of the second occluding portion and is connected to the conveying and cutting device, and the first auxiliary line is provided with more than one clamping part, the clamping parts are used to pass through the second occluding portion through the limiting member under an action of external force, and clamp with the limiting member when the external force disappears.

The present invention has at least the following beneficial effects: the present invention provides an occluder, the occluder comprises a first occluding portion and a second occluding portion, the first occluding portion and the second occluding portion are connected by a connecting portion; the first occluding portion and the second occluding portion are both composed of a mesh structure, the second occluding portion is provided with a lacing member, the lacing member is arranged in a surrounding manner and passes through the mesh at the end of the second occluding portion in sequence, and a limiting member is provided on the lacing member.

The occluder provided by the present invention reduces the inner diameter of the channel formed at the end of the second occluding portion by providing a lacing member and a limiting member, thereby solving the problem of poor locking effect between the prior art occluder and the metal ring, so as to improve the locking success rate of the occluder during surgery. At the same time, the end of the second occluding portion is bound by the lacing member, which increases the structural strength of the end of the second occluding portion, increases the stability of the channel formed at the end of the second occluding portion, and avoids deformation of the end of the second occluding portion, thereby affecting the locking effect.

The present invention also provides an occluder manufacturing device, which is used to manufacture the above-mentioned occluder, comprising a columnar body, wherein a first end of the columnar body is provided with a plurality of first positioning members (A1₁, A1₂...A1ₙ) and a plurality of second positioning members (A2₁, A2₂...A2ₙ), and the second positioning member at the first end of the columnar body is located between two adjacent first positioning members of the first end of the columnar body, wherein a second end of the columnar body is also provided with a plurality of first positioning members (B1₁, B1₂...B1ₙ) and a plurality of second positioning members (B2₁, B2₂...B2ₙ); the second positioning member at the second end of the columnar body is located between two adjacent first positioning members of the second end of the columnar body; the occluder manufacturing device is suitable for an occluder weaving method for manufacturing the occluder above, the occluder weaving method comprises: fixing a single wire on A1₁, and passing the single wire through all the first positioning members and the second positioning members in the order of A1₁, B1₁, A2₁, B2₁...A1ₙ, B1ₙ, A2ₙ, B2ₙ to form a weaved body, wherein the single wire located between the first end of the columnar body and the second end of the columnar body is wound along the outer circumference of the columnar body, wherein, during the weaving process, the single wire is interwoven and passed through the weaved paths up and down to form a mesh structure; fixing a middle portion of the weaved body, and squeezing the two ends of the weaved body toward the middle portion of the weaved body to form an occluder having the first occluding portion and the second occluding portion.

The present invention provides an occluder manufacturing device, which is suitable for manufacturing an occluder with a single wire, so that in the manufacturing process of the occluder, there is no need to perform complex twisting or bundling of multiple wires, which simplifies the production process, reduces the production difficulty and cost, and avoids mutual interference between wires. Secondly, by providing the first positioning member and the second positioning member at both the first end and the second end of the columnar body, the single wire passes through all the first positioning members and the second positioning members in sequence through a specific weaving sequence to weave into a cylindrical weaved body. During the weaving process, the positioning member is used to accurately secure the wire, ensure the stability of the wire during the weaving process, avoid the misalignment of the wire during the weaving process, improve the accuracy of the weaved body, and improve the weaving efficiency. At the same time, the structure of the weaved body is stable, and it is not easy to deform, loosen, etc., which helps to improve the durability and service life of the weaved body. The weaver only needs to pass the wire through the positioning members in sequence according to the preset weaving sequence to complete the weaving work, which greatly simplifies the weaving process and reduces the complexity and technical requirements of the operation.

The present invention also provides an occluder system, comprising: the above-mentioned occluder; a conveying and cutting device; and a first auxiliary line, wherein one end of the first auxiliary line is arranged on an end surface of the first occluding portion of the occluder away from the second occluding portion of the occluder, the other end of the first auxiliary line passes through the second occluding portion and passes through the second occluding portion through the limiting member and is connected with the conveying and cutting device, and the first auxiliary line is provided with more than one clamping part, the clamping part is used to pass through the second occluding portion through the limiting member under the action of external force, and clamp with the limiting member when the external force disappears.

The occluder system provided by the present invention, when the first auxiliary line moves in the opposite direction of the intervention direction under the action of external force, at least one clamping part passes through the second occluding portion through the limiting member. When the occluder is in stable contact with the patent foramen ovale defect and the external force disappears, the first auxiliary line is clamped with the limiting member through the clamping part, so that the occluder remains in a locked state, thereby completing the occlusion of the patent foramen ovale defect. The present invention realizes the locking of the occluder by the cooperation of the clamping part and the limiting member, without using the metal ring in the prior art to lock the occluder, thus avoiding the displacement of the occlude. Compared with the prior art, the present invention provides a new occluder locking structure, which realizes the locking of the occluder by the cooperation of the limiting member and the clamping part on the first auxiliary line. The operation steps of the first auxiliary line are simple, which simplifies the locking steps of the occlude. And the first auxiliary line can be made of degradable materials, which reduces the impact of the metal ring made of non-degradable materials on the human body.

Optionally, in some embodiments, in order to solve the problem that the occluder in the prior art cannot be retracted after the umbrella body is formed, the present invention provides a retrievable occluder system, comprising: an occluder, having a tubular third section and a first section and a second section capable of switching between tubular and disc-shaped forms, the two ends of the third section are respectively connected to the first section and the second section; a connecting mechanism, a recovery tube and a connecting tube are sequentially sleeved from the inside to the outside, and the recovery tube can move along the length direction of the connecting mechanism; an operating mechanism, having a gripping member that can drag the recovery tube to move; a molding auxiliary line, one end of the molding auxiliary line is fixed to one end of the second section away from the third section, and the other end of the molding auxiliary line is fixed to the operating mechanism; one end of the connecting tube is fixed to the operating mechanism, and the other end of the connecting tube is detachably connected to the occluder.

Optionally, the present invention also provides a retrievable occluder system, comprising: an occluder having a tubular third section and a first section and a second section capable of switching between tubular and disc-shaped forms, the two ends of the third section respectively connecting the first section and the second section; a molding auxiliary line, one end of the molding auxiliary line is fixed to an end of the second section away from the third section, and the other end of the molding auxiliary line is fixed to an operating mechanism, the molding auxiliary line is used to tighten the first section and the second section; a withdrawal line, the withdrawal line is wound around an end of the first section away from the third section, the two ends of the withdrawal line are fixed to a connecting tube, and the withdrawal line is used to withdraw the occluder under the action of an external force.

Compared with the prior art, the retrievable occluder system in the present invention comprises an occluder, a connecting mechanism, an operating mechanism, and a molding auxiliary line. When the occluder is delivered to the defective part of the heart, the molding auxiliary line is pulled to squeeze the occluder on the connecting structure, thereby causing the second section to begin to deform from a tubular shape to a disc shape, and the first section also begins to deform from a tubular shape to a disc shape, and passes through a closed end of the first section of the occluder away from the third section through the elastic knot on the molding auxiliary line, so that the occluder remains in a locked state, and both sides of the defective part of the heart are blocked. When the occluder needs to be retracted during the surgery, the gripping member can be moved, and the gripping member drives the recovery tube to move so that the recovery tube passes through the first section and the third section in sequence and presses against the end of the second section away from the third section. Under the action of the recovery tube, the first section and the second section return from a disc shape to a tubular shape. Finally, the entire occluder drags the molding auxiliary line by the doctor to unfold the first section and the second section in the correct position. The implementation of the recyclable occluder system disclosed in the present invention can solve the problem that the occluder cannot be retracted after the umbrella body is formed.

### BRIEF DESCRIPTION OF DRAWINGS

In order to more clearly illustrate the technical solutions in the embodiments of the present invention, the following will briefly introduce the drawings required for use in the description of the embodiments. Obviously, the drawings described below are only some embodiments of the present invention. For those ordinary skilled in the art, other drawings can be obtained based on these drawings without creative work.
Figure 1 is a first structural schematic diagram of the occluder provided in this embodiment;
Figure 2 is a structural schematic diagram of the second occluding portion provided in this embodiment;
Figure 3 is a structural schematic diagram of the first occluding portion provided in this embodiment;
Figure 4 is a structural schematic diagram of the lacing member and the limiting member provided in this embodiment;
Figure 5 is a structural schematic diagram of the occluder manufacturing device provided in this embodiment;
Figure 6 is a structural schematic diagram of the weaved body formed by the occluder manufacturing device provided in this embodiment;
Figure 7 is a structural schematic diagram of the occluder system provided in this embodiment;
Figure 8 is a partial enlarged view of the A area in Figure 7;
Figure 9 is a second structural schematic diagram of the occluder system provided in this embodiment;
Figure 10 is a third structural schematic diagram of the occluder system provided in this embodiment;
Figure 11 is a fourth structural schematic diagram of the occluder system provided in this embodiment;
Figure 12 is a partial enlarged view of the B area in Figure 11;
Figure 13 is a fifth structural schematic diagram of the occluder system provided in this embodiment;
Figure 14 is a partial enlarged view of the C area in Figure 13;
Figure 15 is a sixth structural schematic diagram of the occluder system provided in this embodiment;
Figure 16 is a seventh structural schematic diagram of the occluder system provided in this embodiment;
Figure 17 is an eighth structural schematic diagram of the occluder system provided in this embodiment;
Figure 18 is a ninth structural schematic diagram of the occluder system provided in this embodiment;
Figure 19 is a tenth structural schematic diagram of the occluder system provided in this embodiment;
Figure 20 is an eleventh structural schematic diagram of the occluder system provided in this embodiment;
Figure 21 shows an entirety structural schematic diagram of the overall structure of an occluder in the present invention after it is unfolded into a disc shape;
Figure 22 shows a left view of an occluder in the present invention after it is unfolded into a disc shape;
Figure 23 shows a schematic diagram of a state in which the occluder and the molding auxiliary line and the recovery line are connected in the present invention;
Figure 24 shows a structural schematic diagram of a recyclable occluder system removing the structures near the occluder end in the present invention;
Figure 25 shows an exploded schematic diagram of the operating mechanism in a retrievable occluder system in the present invention;
Figure 26 shows a schematic cross-section diagram of a retrievable occluder system in the present invention after the occluder is removed;
Figure 27A shows a partial enlarged view of area A in Figure 26 (state before cutting line);
Figure 27B shows a partial enlarged view of area A in Figure 26 (state before cutting line);
Figure 28 shows a schematic cross-section diagram of a retrievable occluder system in the present invention;
Figure 29 shows a partial enlarged view of area B in Figure 28; and
Figure 30 shows a partial enlarged view of area C in Figure 28.

Reference signs:
10-occluder; 11-first occluding portion; 12-second occluding portion; 13-connecting portion; 14-lacing member; 15-limiting member; 151-limiting segment; 20-occluder manufacturing device; 21-columnar body; 22-wire; 23-weaved body; 211-first end of columnar body; 212-second end of columnar body; 231-middle portion of weaved body; 232-end of weaved body; 2111-first positioning member of first end of columnar body; 2112-second positioning member of first end of columnar body; 2121-first positioning member of second end of columnar body; 2122-second positioning member of second end of columnar body; 30-first auxiliary line; 31-clamping part; 311-first end of the clamping part; 312-second end of the clamping part; 313-recessed portion; 40-second auxiliary line; 50-conveying and cutting device; 51-first tube body; 52-second tube body; 53-third tube body; 54-conveying member; 55-first cutting member; 56-handle; 511-end cap; 521-first identification member; 522-second identification member; 523-third identification member; 551-driving member; 552-cutting head; 5111-channel; 60-delivery sheath; 70-loader; third section-110; first section-120; second section-130; connecting mechanism-230; recovery tube-210; connecting tube-220; operating mechanism-300; gripping member-310; molding auxiliary line-420; node-410; closed end-140; winding assembly-320; line-restraining tube-230; recovery line-500; cutting line tube-700; first through hole-2210; withdrawal line-80; heat shrink tube-90; locking switch-330; fixing block-340; cutting-line switch-350; second through hole-2310; rigid section-2220; flexible section-2230; second cutting member-710; sheath tube-100.

### DETAILED DESCRIPTION OF EMBODIMENTS

Various embodiments of the present invention hereinafter will be described in more detail. The present invention may have various embodiments, and adjustments and changes may be made therein. It should be noted that in the present invention, unless otherwise clearly specified and defined, terms such as "installation", "connection", and "fixation" should be understood in a broad sense, for example, it may be a fixed connection, a detachable connection, or an integral connection; it may be a mechanical connection or an electrical connection; it may be a direct connection or an indirect connection through an intermediate medium; it may be a communication between two elements. For those ordinary skilled in the art, the specific meanings of the above terms in the present invention can be understood according to the specific situations.

In the present invention, those ordinary skilled in the art need to understand that the terms indicating the orientation or positional relationship in the text are based on the orientation or positional relationship shown in the drawings, which is only for the convenience of describing the present invention and simplifying the description, and do not indicate or imply that the device or element referred to must have a specific orientation, be constructed and operated in a specific orientation, and therefore cannot be understood as a limitation on the present invention.

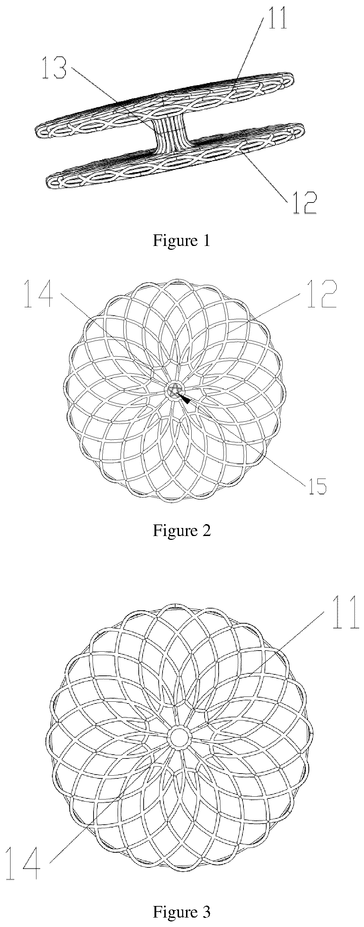

As shown in Figures 1 to 4, in an embodiment of the present invention, an occluder 10 is provided, the occluder 10 includes a first occluding portion 11, a second occluding portion 12 and a connecting portion 13 located between the first occluding portion 11 and the second occluding portion 12, the first occluding portion 11 and the second occluding portion 12 are both composed of a mesh structure, the second occluding portion 12 is provided with a lacing member 14. The lacing member 14 is arranged in a surrounding manner and passes through the mesh at the end of the second occluding portion 12 in sequence, and a limiting member 15 is provided on the lacing member 14.

One of the functions of the limiting member 15 is to reduce the inner diameter of the lacing member 14, so as to prevent the fasteners used for locking the auxiliary line from entering the second occluding portion 12 through the lacing member 14, resulting in poor locking effect of the occluder 10 or even locking failure, thereby improving the success rate of locking. The occluder 10 is made of completely degradable materials, that is, the first occluding portion 11, the second occluding portion 12, the connecting portion 13, the lacing member 14 and the limiting member 15 are all made of completely degradable materials.

A traditional occluder is provided with a metal mortise, and is connected to the delivery system through the metal mortise, but the metal mortise cannot be degraded after entering the human body. The present embodiment does not provide a metal mortise, and the occluder 10 is made of a completely degradable material, which overcomes the defects of the traditional occluder and is less harmful to the human body. In addition, the installation process of the traditional metal mortise and the second occluding portion is complicated. The present embodiment does not provide a metal mortise, which reduces the production difficulty of the occluder 10, thereby improving the production efficiency of the occluder 10.

The occluder 10 provided in the present embodiment reduces the inner diameter of the channel formed at the end of the second occluding portion 12 by providing a lacing member 14 and a limiting member 15, so as to improve the success rate of locking of the occluder 10 during surgery. At the same time, the end of the second occluding portion 12 is bounded by the lacing member 14, which increases the structural strength of the end of the second occluding portion 12, increases the stability of the channel formed at the end of the second occluding portion 12, and avoids deformation of the end of the second occluding portion 12, thereby affecting the locking effect.

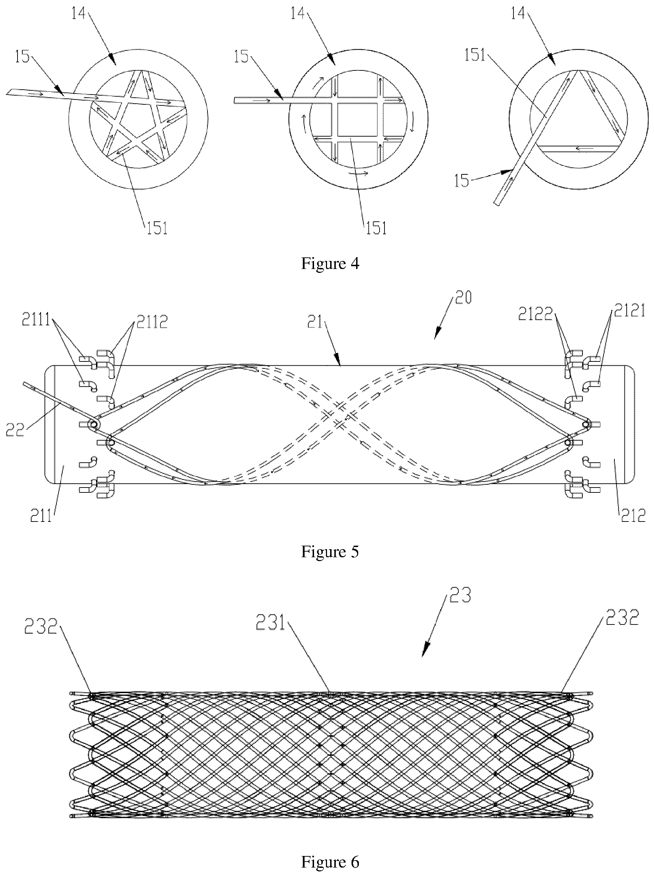

As shown in Figure 6, in an embodiment of the present invention, a weaved body 23 with a mesh structure in a cylindrical shape is formed by weaving a wire 22. Then the weaved body 23 is pressed to form an occluder 10, wherein the two ends of the weaved body 23 with a mesh structure in a cylindrical shape are squeezed toward the middle to form a first occluding portion 11 and a second occluding portion 12 protruding from the middle portion 231 of the weaved body, and the middle portion 231 of the weaved body forms a connecting portion 13.

As shown in Figure 1 to Figure 4, in an embodiment of the present invention, a lacing member 14 is provided at the end of the second occluding portion 12 away from the first occluding portion 11, and the diameter of the lacing member 14 is smaller than the diameter of the wire 22. The present invention uses a lacing member with a diameter smaller than the diameter of the wire to secure the end of the second occluding portion, so that the outer diameter of the end of the occluder is reduced when the occluder is in a retracted state. During the delivery process, the end of the occluder first contacts the surrounding tissues such as the blood vessel wall. The smaller diameter of the end can reduce the pressure and damage to the surrounding tissues, reduce the risk of complications such as bleeding and hematoma, and can improve the safety of the operation to a certain extent, and reduce additional damage to the patient's body. At the same time, the pain and discomfort of the patient during the postoperative recovery process will be reduced accordingly, which will help shorten the patient's recovery time and improve the recovery efficiency.

Secondly, when the occluder passes through narrow channels such as blood vessels, the end with a smaller diameter can reduce the contact area with the blood vessel wall, thereby reducing friction resistance and resistance during delivery, making it easier for the occluder to be delivered to the target location. The occluder with a smaller outer diameter at the end makes the occluder have better flexibility at the bends of the blood vessels, and can pass and adapt to these structures more easily, reducing the surgical risks caused by delivery difficulties. In addition, it is particularly conducive to tissue endothelialization.

Specifically, the wire uses a 3-0 wire with a diameter of 0.249mm, the opening gathering wire uses a 4-0 wire with a diameter of 0.2mm, and the limiting member uses a 5-0 wire with a diameter of 0.1mm. The lacing member 14 is arranged in a surrounding manner and passes through each mesh of the end of the second occluding portion 12 away from the first occluding portion 11 in sequence.

In this embodiment, the lacing member 14 is arranged at the end of the second occluding portion 12 away from the first occluding portion 11 to reduce the inner diameter of the end of the second occluding portion 12 away from the first occluding portion 11, so as to improve the locking success rate of the occluder 10. At the same time, the end of the second occluding portion 12 away from the first occluding portion 11 is bound by the lacing member 14, which increases the structural strength of the end of the second occluding portion 12, increases the stability of the second occluding portion 12, and avoids deformation of the end of the second occluding portion 12, thereby affecting the locking effect.

The end of the first occluding portion 11 away from the second occluding portion 12 is also provided with a lacing member 14, which is arranged in a surrounding manner and passes through each mesh of the end of the first occluding portion 11 away from the second occluding portion 12 in sequence. In this embodiment, the end of the first occluding portion 11 composed of a mesh structure is bound by the lacing member 14, which increases the structural strength of the end of the first occluding portion 11, increases the stability of the first occluding portion 11, and avoids the deformation of the first occluding portion 11, thereby affecting the occluding effect.

As shown in Figure 1 to Figure 4, in the embodiment of the present invention, a limiting member 15 is provided on the lacing member 14 located at the end of the second occluding portion 12 away from the first occluding portion 11. The limiting member 15 includes a plurality of limiting segments 151, and the plurality of limiting segments 151 are connected and enclosed on the lacing member 14 to form a closed pattern. In this embodiment, the limiting member 15 is provided to further reduce the inner diameter of the end channel of the second occluding portion 12 away from the first occluding portion 11, thereby improving the locking effect. The stability of the occluder 10 is further improved when it is in a locked state, and it is ensured that the occluder 10 is in a locked state for a long time to block the defect of the patent foramen ovale.

The diameter of the limiting member 15 is smaller than the diameter of the lacing member 14. The starting end and the ending end of the limiting member 15 are fixed on the lacing member 14 by tying a knot. In the embodiment of the present invention, a plurality of limiting segments 151 are sequentially connected and enclosed on the lacing member 14 to form a closed pattern.

In the embodiment of the present invention, the lacing member 14 has a plurality of winding positions, the starting end of the limiting member 15 is fixed on the lacing member 14, and the ending end of the limiting member 15 is fixed on the lacing member 14 after passing through a plurality of winding positions in sequence. The limiting member 15 located between the two winding positions is the limiting segment 151. In this embodiment, the limiting member 15 is wound on the lacing member 14 to form a plurality of limiting segments 151, which simplifies the installing steps of the limiting member 15 and the lacing member 14.

In the embodiment of the present invention, a plurality of limiting segments 151 are connected and enclosed on the lacing member 14 to form any one of a star shape, a tic-tac-toe shape or a triangle. This embodiment makes the limiting member 15 contact each point on the lacing member 14 according to the limited pattern, simplifies the difficulty of arranging the limiting member 15, makes the limiting member 15 easy to install, and reduces the inner diameter of the limiting member 15, thereby improving the clamping effect.

In another embodiment of the present invention, the limiting member 15 includes an enclosure and two or more extensions, the enclosure is connected end to end to form a closed pattern, one end of the extension is arranged on the enclosure, and the other end extends away from the enclosure and is arranged on the lacing member 14, and the two or more extensions are arranged at intervals along the circumference of the enclosure to fix the enclosure on the lacing member 14. The closed pattern formed by the enclosure connected end to end is a circular, elliptical, polygonal or irregular closed pattern.

As shown in Figure 5, in an embodiment of the present invention, an occluder manufacturing device 20 is provided for manufacturing the above-mentioned occluder 10. The occluder manufacturing device 20 comprices a columnar body 21. A first end 211 of the columnar body is provided with a plurality of first positioning members 2111 (A1₁, A1₂...A1ₙ) and a plurality of second positioning members 2112 (A2₁, A2₂...A2ₙ). The second positioning member 2112 of the first end of the columnar body is located between two adjacent first positioning members 2111 of the first end of the columnar body. The second end 212 of the columnar body is also provided with a plurality of first positioning members 2121 (B1₁, B1₂...B1ₙ) and a plurality of second positioning members 2122 (B2₁, B2₂...B2ₙ). The second positioning member 2122 of the second end of the columnar body is located between two adjacent first positioning members 2121 of the second end of the columnar body.

The columnar body 21 is a plastic tube or metal tube with a smooth surface to reduce the friction between the wire 22 and the columnar body 21 and prevent the surface of the columnar body 21 from rubbing against the wire 22. A plurality of first positioning members 2111 are arranged circumferentially along the first end 211 of the columnar body, and a plurality of second positioning members 2112 are arranged circumferentially along the first end 211 of the columnar body. A plurality of first positioning members 2121 are arranged circumferentially along the second end 212 of the columnar body, and a plurality of second positioning members 2122 are arranged circumferentially along the second end 212 of the columnar body.

As shown in Figure 5 and Figure 6, the occluder manufacturing device 20 provided in this embodiment is suitable for an occluder weaving method to form a weaved body for manufacturing the above-mentioned occluder 10. The occluder weaving method includes: fixing a single wire 22 on A1₁, and passing through all the first positioning members and the second positioning members in the order of A1₁, B1₁, A2₁, B2₁...A1ₙ, B1ₙ, A2ₙ, B2ₙ to form a weaved body 23. The single wire 22 located between the first end 211 of the columnar body and the second end 212 of the columnar body is wound along the outer periphery of the columnar body 21. During the weaving process, the single wire 22 is interwoven and passes through the weaved paths up and down to form a mesh structure. The middle portion 231 of the weaved body is fixed, and the two ends 232 of the weaved body is squeezed toward the middle portion of the weaved body 23 to form an occluder 10 having a first occluding portion 11 and a second occluding portion 12.

The occluder manufacturing device 20 provided in this embodiment is suitable for manufacturing the occluder 10 with a single wire 22, so that in the manufacturing process of the occluder 10, it is not necessary to twist or bundle multiple wires in a complicated manner, which simplifies the production process, reduces the production difficulty and cost, and avoids mutual interference between wires. Secondly, by providing the first positioning member and the second positioning member at both the first end 211 and the second end 212 of the columnar body, the wire 22 passes through all the first positioning members and the second positioning members in sequence in a specific weaving sequence to weave into a cylindrical weaved body 23. During the weaving process, the positioning member is used to accurately fix the wire 22, ensure the stability of the wire 22 during the weaving process, avoid the misalignment of the wire 22 during the weaving process, improve the accuracy of the weaved body 23, and improve the weaving efficiency. At the same time, the structure of the weaved body 23 is stable, and it is not easy to deform, loosen, etc., which helps to improve the durability and service life of the weaved body 23. The weaver only needs to pass the wire 22 through the positioning members in sequence according to the preset weaving sequence to complete the weaving work, which greatly simplifies the weaving process and reduces the complexity and technical requirements of the operation.

One end 232 of the weaved body formed by the occluder manufacturing device provided by this embodiment is squeezed toward the middle portion of the weaved body 23 to form the second occluding portion 12, and the opening of the cylindrical weaved body end 232 is squeezed to form a channel at the end of the second occluding portion 12. The occluder manufacturing device provided by this embodiment simplifies the subsequent processing steps of the weaved body 23, so that the weaved body 23 can form a channel at the end of the second occluding portion 12 after being squeezed, thereby reducing the processing steps of the occluder 10.

In the embodiment of the present invention, a detachable connection structure (not shown in the figure) is provided between at least one of the first positioning member and the second positioning member and the columnar body 21 to adjust the distance between the two adjacent first positioning members and the distance between the two adjacent second positioning members. This allows the weaver to adaptively adjust the distance between the two adjacent first positioning members and the distance between the two adjacent second positioning members according to the size and density of the weaved body actually required, thereby increasing the applicable scope of the occluder manufacturing device. The detachable connection structure includes one of a plug-in connection structure, a snap-fit connection structure and a magnetic connection structure, but is not limited to this.

As shown in Figure 5, in an embodiment of the present invention, a sliding connection structure (not shown in the figure) is provided between at least one of the first positioning member and the second positioning member and the columnar body 21 to adjust the distance between adjacent first positioning members and second positioning members. This allows the weaver to adaptively adjust the distance between adjacent first positioning members and second positioning members according to the size and density of the weaved body actually required, thereby increasing the applicable scope of the occluder manufacturing device.

Optionally, the first positioning member and the second positioning member are hook-shaped. Optionally, the distance between two adjacent first positioning members is between 1mm and 10mm. Optionally, the distance between two adjacent second positioning members is between 1mm and 10mm. The distance between adjacent first positioning member and second positioning member is between 2mm and 10mm. Optionally, the height of the first positioning member is between 1mm and 10mm, and the height of the second positioning member is between 1mm and 10mm.

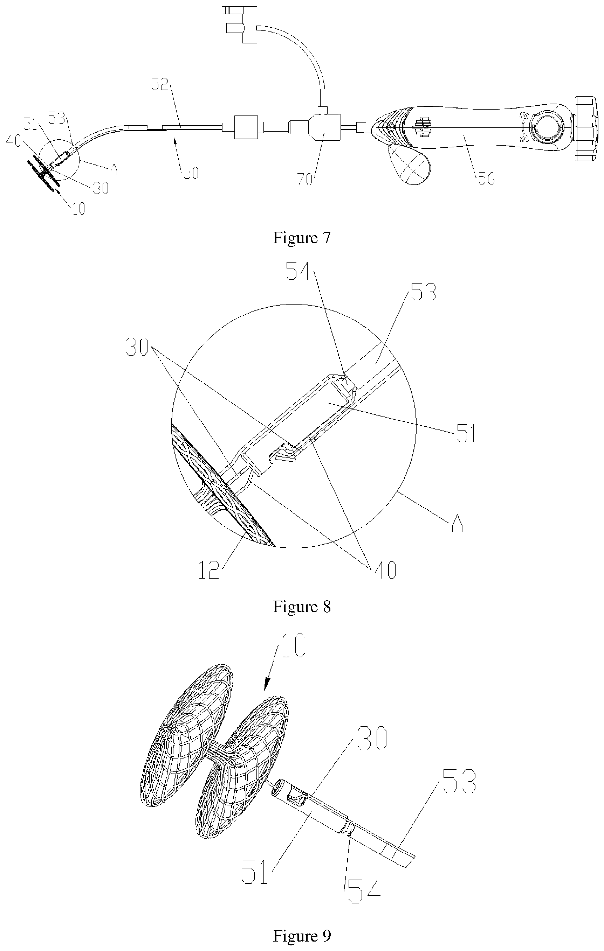

As shown in Figure 1 to Figure 4 and Figure 7 to Figure 20, in an embodiment of the present invention, an occluder system is provided, comprising: the above-mentioned occluder 10, a conveying and cutting device 50, and a first auxiliary line 30. One end of the first auxiliary line 30 is arranged on an end surface of the first occluding portion 11 of the occluder away from the second occluding portion 12 of the occluder, the other end of the first auxiliary line 30 passes through the second occluding portion 12 and passes through the second occluding portion 12 through the limiting member 15 to connect with the conveying and cutting device 50. The first auxiliary line 30 is provided with one or more clamping parts 31, which are used to pass through the second occluding portion 12 through the limiting member 15 under the action of external force, and clamp with the limiting member 15 when the external force disappears.

The occluder system provided in this embodiment is such that the first auxiliary line 30 moves in the opposite direction of the intervention direction under the action of an external force, while at least one clamping part 31 passes through the second occluding portion 12 through the limiting member 15. When the occluder 10 is in stable contact with the patent foramen ovale defect, the first auxiliary line 30 is clamped with the limiting member 15 through the clamping part 31 to keep the occluder 10 in a locked state and block the patent foramen ovale defect, and then the conveying member 54 is driven to move by the handle 56 to cut off the first auxiliary line 30 in the first tube body 51 to complete the occlusion operation. This embodiment achieves the locking of the occluder 10 through the cooperation of the clamping part 31 and the limiting member 15, thereby avoiding the displacement of the occluder 10. Compared with the prior art, this embodiment provides a new occluder locking structure, which achieves the locking of the occluder 10 through the cooperation of the clamping part 31 on the first auxiliary line 30 and the limiting member 15. The operation steps of the first auxiliary line 30 are simple, which simplifies the locking steps of the occluder 10. The first auxiliary line 30 can be made of completely degradable materials, reducing the impact of non-degradable materials on the human body.

Another traditional occluder cooperates with a metal ring to complete the locking of the occluder, but the metal ring cannot be degraded after being inserted into the human body. This embodiment overcomes the defects of the traditional occluder system, and completes the locking of the occluder by cooperating with the occluder 10 made of completely degradable materials and the first auxiliary line 30, so that the occluder 10 and the first auxiliary line 30 inserted into the human body have completely degradable functions, which are less harmful to the human body.

As shown in Figures 1-4 and Figures 7-20, in the embodiments of the present invention, the occluder system also includes: a second auxiliary line 40 connected to the second occluding portion 12, which is used to drive the occluder 10 to move under the action of external force; and the conveying and cutting device 50 connected in advance with the occluder 10 through the first auxiliary line 30 and the second auxiliary line 40. In the occluder system provided in this embodiment, the occluder 10 and the conveying and cutting device 50 are connected by the first auxiliary line 30 and the second auxiliary line 40, so that the occluder 10 and the conveying and cutting device 50 are pre-installed. Medical staff do not need to spend time connecting the occluder 10 and the conveying and cutting device 50 during the operation, which saves operation time and improves the convenience of use.

The occluder system provided in this embodiment realizes the locking of the occluder 10 through the first auxiliary line 30, and drives the first occluding portion 11 and the second occluding portion 12 to withdraw through the second auxiliary line 40 when the occluder 10 is displaced, the occluder 10 cannot completely close the patent foramen ovale defect, or the operation fails. The present invention only provides with two auxiliary lines to achieve the locking and withdrawal of the occluder 10, and the structure is simple. Compared with the prior art, the present invention does not need to provide multiple operation lines, which simplifies the structure of the occluder system and the surgical operation process. The first auxiliary line 30 and the second auxiliary line 40 have the same moving direction, which will not cause confusion of the first auxiliary line 30/the second auxiliary line 40, nor will it cause the first auxiliary line 30/the second auxiliary line 40 to be knotted. The operation steps of the first auxiliary line 30 and the second auxiliary line 40 are simple, which simplifies the operation steps of the occluder.

As shown in Figure 1 to Figure 4 and Figure 7 to Figure 20, in the embodiment of the present invention, the conveying and cutting device 50 comprises a first tube body 51, a second tube body 52, a third tube body 53, a conveying member 54, a first cutting member 55 and a handle 56. One end of the conveying member 54 is fixed to the handle 56 and passes through the second tube body 52 and the third tube body 53 in sequence and then connected to the first tube body 51. The first auxiliary line 30 extends into the end of the first tube body 51 into the first tube body, exits through the side wall of the first tube body 51 and then passes through the first tube body 51, and is located between the conveying member 54 and the second tube body 52. The second auxiliary line 40 enters from the end of the first tube body 51, exits through the first tube body 51 from the side wall of the first tube body 51, and is located between the conveying member 54 and the third tube body 53. One end of the first cutting member is arranged on the handle, and the other end passes through the second tube body and the third tube body in sequence and is located in the first tube body. The first cutting member 55 is used to move relative to the first tube body 51 under the drive of the handle 56 to cut the first auxiliary line 30 and the second auxiliary line 40.

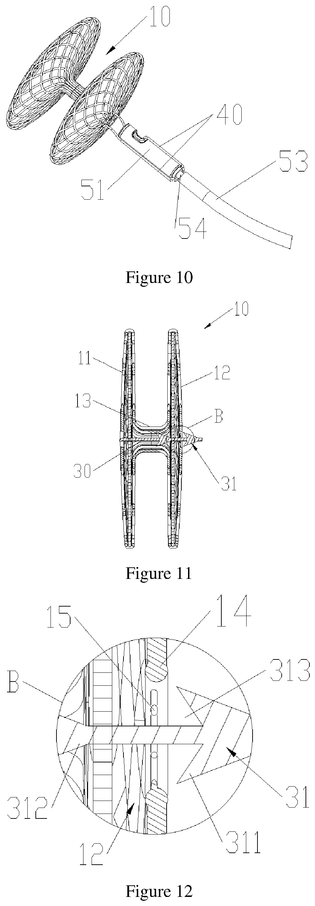

As shown in Figure 11 and Figure 12, in the embodiment of the present invention, the number of the clamping parts 31 is more than two, and the more than two clamping parts 31 are sequentially arranged on the first auxiliary line 30. Before the first auxiliary line 30 is not subjected to external force, the clamping parts 31 are located in at least one of the first occluding portion 11, the connecting portion 13 and the second occluding portion 12. The distance between the first clamping part 31 and the last clamping part 31 is greater than the distance between the first occluding portion 11 and the second occluding portion 12 after they are completely retracted. The occluder 10 is made of elastic material, and the external force applied to the first auxiliary line 30 to make the clamping part 31 pass through the limiting member 15 is greater than the force required for the second occluding portion 12 to produce elastic deformation. The reset force applied by the first occluding portion 11 to the first auxiliary line 30 is less than the force required for the second occluding portion 12 to produce elastic deformation.

As shown in Figure 11 to Figure14, in the embodiment of the present invention, the first end 311 of the clamping part is closer to the first occluding portion 11 than the second end 312 of the clamping part. The distance between the outer wall of the first end 311 of the clamping part and the first auxiliary line 30 is greater than the distance between the outer wall of the second end 312 of the clamping part and the first auxiliary line 30, so that the clamping part 31 passes through the limiting member 15 under the action of external force and is clamped with the limiting member 15 through the first end 311 of the clamping part.

In this embodiment, by providing the first end 311 and the second end 312 of the clamping part of different sizes, when the clamping part 31 passes through the limiting member 15, the second end 312 of the clamping part can easily pass through the limiting member 15, and only the first end 311 of the clamping part applies a squeezing force to the inner wall of the limiting member 15, thereby reducing the friction force on the inner wall of the limiting member 15 and reducing the wear of the inner wall of the limiting member 15 by the first end 311 of the clamping part.

In the embodiment of the present invention, the angle between the outer wall of the clamping part 31 and the first auxiliary line 10 is between 10° and 80°. In this embodiment, the first end 311 of the clamping part gradually decreases toward the second end 312 of the clamping part, so as to avoid the uneven outer wall of the clamping part 31 from causing wear on the inner wall of the limiting member 15, thereby avoiding the outer wall of the clamping part 31 from affecting the limiting function of the limiting member 15. The cross-section of the clamping part 31 is triangular, quasi-triangular, trapezoidal, quasi-trapezoidal, V-shaped or quasi-V-shaped, but not limited thereto.

In the embodiment of the present invention, there is an arc surface between the outer wall of the first end 311 of the clamping part and the outer wall of the second end 312 of the clamping part to reduce the wear of the clamping part 31 on the limiting member 15. The angle between the connecting line between the outer wall of the first end 311 of the clamping part and the outer wall of the second end 312 of the clamping part and the first auxiliary line 10 is between 10° and 80°.

As shown in Figure 11 to Figure 14, in the embodiment of the present invention, the first end 311 of the clamping part is recessed toward the second end 312 of the clamping part to form a recessed portion 313, and the angle between the side wall of the recessed portion and the first auxiliary line 30 is between 0° and 75°. The recessed portion 313 is used to accommodate the lacing member 14 and/or the closed pattern formed by the multiple limiting segments 151 when the clamping part 31 is clamped with the limiting member 15.

In this embodiment, a recessed portion 313 is provided to accommodate the lacing member 14 forming the limiting member 15 and/or to accommodate the closed pattern or part the of closed pattern forming the limiting member 15, so that the first end 311 of the clamping part is away from the lacing member 14 and/or the closed pattern, thereby avoiding the failure of the clamping part 31 and the limiting member 15 to be clamped. The inner diameter of the recessed portion 313 is larger than the outer diameter of the closed pattern enclosed by multiple limiting segments 151. The inner diameter of the recessed portion 313 is larger than the outer diameter of the lacing member 14.

As shown in Figure 11 to Figure 14, in this embodiment, the first end 311 of the clamping part is recessed toward the second end 312 of the clamping part to form a recessed portion 313, and the recessed portion 313 makes the first end 311 of the clamping part sharp-pointed. In this embodiment, the first end 311 of the clamping part is sharp-pointed by the recessed portion 313, so that when the clamping part 31 is clamped with the limiting member 15, the first end 311 of the clamping part can enter the second occluding portion 12 through the gap between the mesh or closed pattern of the second occluding portion 12 and the lacing member 14.

As shown in Figure 11 to Figure 14, in the embodiment of the present invention, the clamping part 31 is in the shape of a fishbone barb, so that when the clamping part 31 is clamped with the limiting member 15, the barb of the clamping part 31 can enter the second occluding portion 12 through the gap between the mesh or closed pattern of the second occluding portion 12 and the lacing member 14, so as to increase the clamping stability of the clamping part 31 and the limiting member 15.

The occluder system provided in this embodiment is configured to be used together with the delivery sheath 60. The occluder 10 is located in the delivery sheath 60. The medical staff brings the first tube body 51 to move by pushing the conveying member 54 to move, so that the first occluding portion 11 and the second occluding portion 12 move to expose the delivery sheath 60.

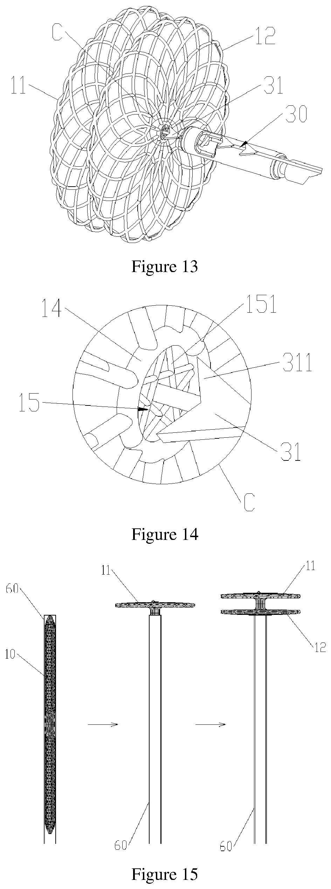

As shown in Figure 15, the first occluding portion 11 and the second occluding portion 12 both have a retracted state in the delivery sheath 60 and a released state outside the delivery sheath 60. In the embodiment of the present invention, the occluder system also includes a loader 70. When in use, the occluder 10 is first pre-installed in the loader 70. The loader 70 is communicated with the delivery sheath 60, and the second tube body 52 is pushed toward the distal end of the occluder system. The occluder 10 is used to move in the loader 70 and move into the delivery sheath 60 under the drive of the second tube body 52.

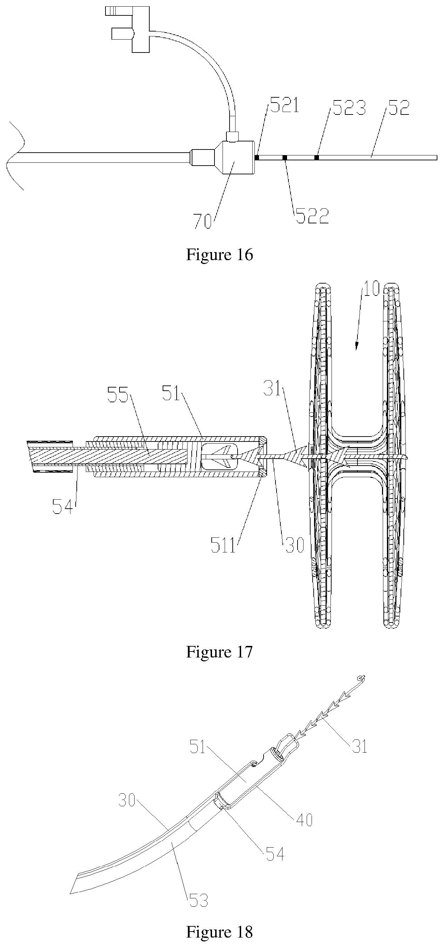

As shown in Figures 15 and 16, in the embodiment of the present invention, at least three identification members are arranged on the second tube body 52, and the at least three identification members include a first identification member 521, a second identification member 522 and a third identification member 523 arranged in sequence. When the occluder system is in use, and the first identification member 521 moves to the proximal end of the loader 70, the first occluding portion 11 and the second occluding portion 12 are both located in the delivery sheath 60, both in a retracted state, and the end of the first occluding portion 11 away from the second occluding portion 12 is located at the distal end of the delivery sheath 60. When the second identification member 522 moves to the proximal end of the loader 70, the first occluding portion 11 is located outside the delivery sheath 60, in a released state, and the second occluding portion 12 is located inside the delivery sheath 60, in a retracted state. When the third identification member 523 moves to the proximal end of the loader 70, the first occluding portion 11 and the second occluding portion 12 are both extended out of the delivery sheath 60, both in a released state.

In this embodiment, medical staff can determine the position and state of the first occluding portion 11 and the second occluding portion 12 by the moving position of the first identification member 521, the second identification member 522 and the third identification member 523, without using X-rays to locate the first occluding portion 11 and the second occluding portion 12, which can reduce the use of X-rays and reduce the impact of X-rays on medical staff and patients.

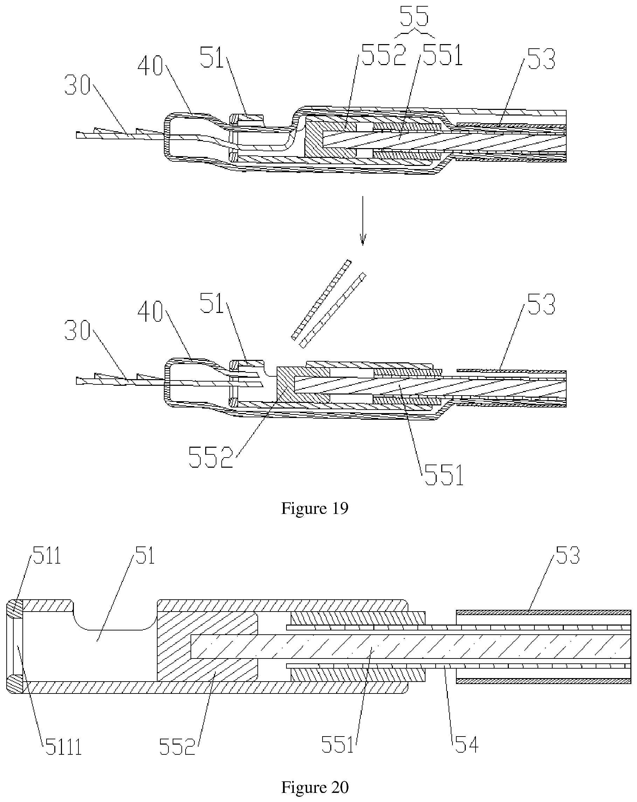

As shown in Figures 17 and 20, in this embodiment, an end cap 511 is provided at the end of the first tube body 51, and a channel 5111 is provided on the end cap 511. The first auxiliary line 30 and the second auxiliary line 40 are both extended into the first tube body 51 through the channel 5111 and exit through the first tube body 51 through the side wall of the first tube body 51. Optionally, providing the end cap 511, the first auxiliary line 30 and the second auxiliary line 40 are prevented from rubbing against the distal end of the first tube body 51, thereby damaging the first auxiliary line and the second auxiliary line. A rounded structure is provided on the side wall of the channel 5111 to reduce the friction of the side wall of the channel 5111 on the first auxiliary line 30 and the second auxiliary line 40.

As shown in Figures 19 and 20, in the embodiment of the present invention, the first cutting member 55 includes a driving member 551 and a cutting head 552, and the cutting head 552 is movably arranged in the first tube body 51. One end of the driving member 551 is movably arranged on the handle 56, and the other end passes through the second tube body 52 and the third tube body 53 in sequence and then extends into the first tube body 51 to connect with the cutting head 552. The driving member 551 is used to move relative to the handle 56 under the drive of the handle 56, thereby driving the cutting head 552 to move relative to the first tube body 51. In the embodiment of the present invention, the third tube body 53 is a heat shrink tube to fix the second auxiliary line 40 on the conveying member 54.

In some prior arts, the patent foramen ovale occluders on the market are mainly metal occluders. However, metal occluders have some disadvantages. For example, metal occluders exist for life after implantation, which makes patients lose the channel for re-interventional treatment of other heart diseases. Long-term existence in the human body may cause some complications. Some people are allergic to nickel-titanium alloys, etc., which makes degradable occluders a current research hotspot.

Degradable occluders can be degraded within a certain period of time, and there is almost no foreign matter left in the heart after degradation. It not only achieves the function of occlusion, but also minimizes the harm to the human body. However, degradable occluders also have some defects that cannot be ignored. First, the shape memory performance of degradable occluders is not as good as that of nickel-titanium alloy occluders. After the degradable occluder passes through the channel to the defect site, it is often a double shuttle shape. This requires that if the degradable occluder wants to achieve the same occlusion effect as the metal occluder, it must assist the molding of the degradable occluder. The current technology is generally: adding the molding auxiliary line 420 assists the molding of the occluder. The molding auxiliary line 420 is fixedly connected to the head end of the umbrella body of the occluder away from the handle end. The other end of the molding auxiliary line 420 is outside the body. The surgeon can tighten the molding auxiliary line 420 outside the body, and the auxiliary line pulls the umbrella body to shape. Generally, an elastic knot is designed on the molding line. The tail end of the umbrella body of the occluder is provided with a hole. The size relationship between the elastic knot and the hole is that the knot is larger than the hole. By pulling the molding auxiliary line 420, the elastic knot on the molding line passes through the hole at the tail end of the umbrella body through elastic deformation, thereby achieving the effect of assisting the umbrella body molding and locking the umbrella body.

At this time, another problem that cannot be ignored arises. Due to the existence of the molding auxiliary line 420 and the elastic knot, the elastic knot cannot be withdrawn after passing through the hole at the tail end of the umbrella body. If during the operation, due to interference from some other factors, it is impossible to withdraw the umbrella body after the umbrella body is formed and locked. The occluder can only be removed by forced pulling, or the occluder can only be removed at the release position of the occluder through surgical operation. This is not an ideal situation for both the surgeon and the patient.

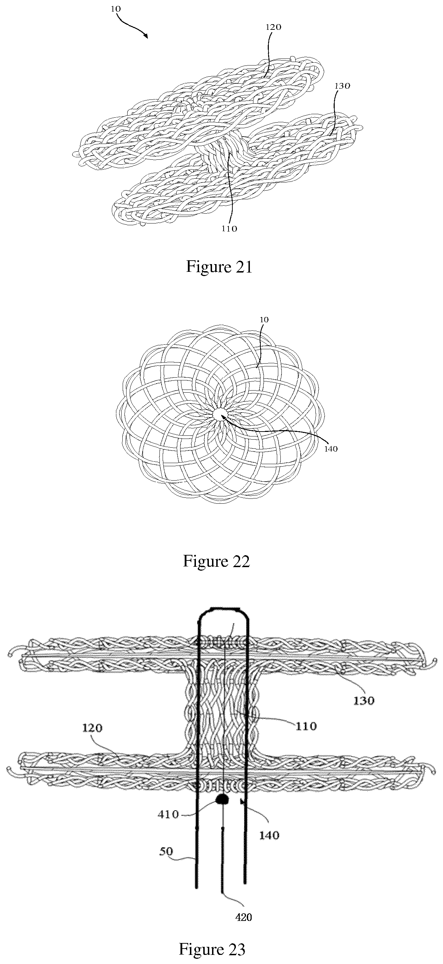

In some embodiments, in order to solve the problem that the occluder 10 cannot be withdrawn after the umbrella body is formed, please refer to Figures 21-30. The present invention provides a retrievable occluder system, including: an occluder 10, having a tubular third section 110 and a first section 120 and a second section 130 that can switch between tubular and disc shapes, and the two ends of the third section 110 are respectively connected to the first section 120 and the second section 130; a connecting mechanism 20, which is sequentially sleeved with a recovery tube 210 and a connecting tube 220 from the inside to the outside, and the recovery tube 210 can move along the length direction of the connecting mechanism 20; an operating mechanism 30, which has a gripping member 310 that can drag the recovery tube 210 to move; a molding auxiliary line 420, one end of which is fixed to the end of the second section 130 away from the third section 110, and the other end is fixed to the operating mechanism 30. One end of the connecting tube 220 is fixed to the operating mechanism 30, and the other end of the connecting tube 220 is detachably connected to the occluder 10.

Compared with the prior art, the retrievable occluder system disclosed in the present invention has an occluder 10, a connecting mechanism 20, an operating mechanism 30 and a molding auxiliary line 420. When the occluder 10 is sent to the heart defect site, the molding auxiliary line 420 is pulled to squeeze the occluder 10 on the connecting mechanism 20, thereby causing the second section 130 to begin to deform from a tubular shape to a disc shape. The first section 120 also begins to deform from a tubular shape to a disc shape, and the elastic knot on the molding auxiliary line passes through the closed end on the first section of the occluder away from the closing of the third section, so that the occluder remains in a locked state, so that both sides of the heart defect site are blocked. When the occluder 10 needs to be withdrawn during the operation, the gripping member 310 can be moved. The gripping member 310 drives the recovery tube 210 to move so that the recovery tube 210 passes through the first section 120 and the third section 110 in sequence and presses against the end of the second section 130 away from the third section 110. Under the action of the recovery tube 210, the first section 120 and the second section 130 return from a disc shape to a tubular shape. Finally, the entire occluder 10 drags the molding auxiliary line 420 by the doctor to deploy the first section 120 and the second section 130 at the correct position. The implementation of the retrievable occluder system in the present invention can solve the problem that the occluder 10 cannot be retrieved after the umbrella body is formed. The doctor then releases the entire occluder 10 to the correct position, and then assists in forming and releasing the occluder 10 by pulling the auxiliary forming line 40.

Generally, a degradable mesh fabric is also provided on the first section 120 and the second section 130 for blocking the foramen ovale, and the first section 120 and the second section 130 are made of degradable materials. Degradable materials include one or two or more copolymers of polylactic acid, poly(L-lactic acid), poly(D,L-lactic acid), poly(glycolic acid), polycaprolactone, polydioxanone, polyhydroxybutyrate, polyanhydride, polyphosphate, polyurethane, polycarbonate, silk protein. Of course, an anticoagulant layer, such as heparin, can also be coated on the occluder 10 to prevent thrombosis and expansion, reduce complications, and reduce patient mortality. A developing coating can also be coated on the occluder 10 to facilitate the doctor to correctly locate the patent foramen ovale during surgery. The recovery tube 210 and the connecting tube 220 are generally made of flexible materials, so that they can be bent.

In some embodiments, please refer to Figure 23, the molding auxiliary line 420 is provided with a node 410 at one end close to the occluder 10, and the node 410 can pass through or be stuck at the outer closed end 140 of the first section 120.

The occluder 10 is initially tubular, and at this time, the node 410 of the molding auxiliary line 420 is inside the second section 130. When the molding auxiliary line 420 is pulled, one end of the molding auxiliary line 420 pulls the two ends of the second section 130 of the molding auxiliary line 420 to start to approach, gradually changing from a tubular shape to a disc shape. The molding auxiliary line 420 continues to be pulled, the node 410 passes through the third section 110 and the first section 120, and the node 410 squeezes the closed end 140 of the first section 120 and drills out from the closed end 140. At this time, the molding auxiliary line 420 is released, and the node 410 is also stuck at the outer closed end 140 of the first section 120. It should be noted that the node 410 can be a structure such as a knot, a fishbone line or a spring fastener. The connection method between the second section 130 and the molding auxiliary line 420 is not unique, and can be knotted, riveted. Alternatively, a line symmetrically passes through the molding auxiliary line 420 to achieve the connection, etc.

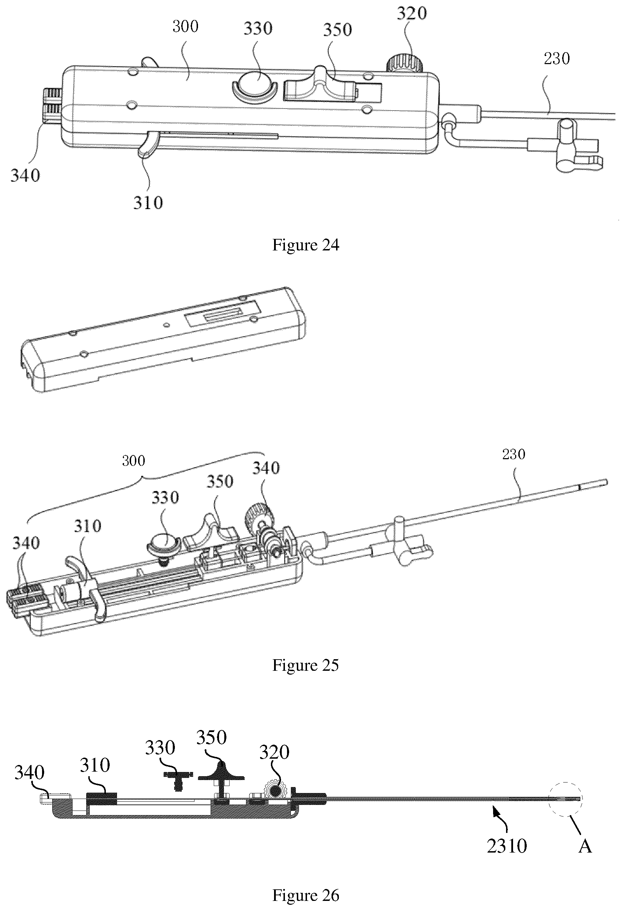

In some embodiments, please refer to Figure 24 to Figure 26, the operating mechanism 30 also includes a winding assembly 320, and the molding auxiliary line 420 is fixed on the winding assembly 320. Rotating the winding assembly 320 can wind the molding auxiliary line 420. In this embodiment, in order to prevent the molding auxiliary line 420 from being pulled by mistake, a winding assembly 320 is provided on the operating mechanism 30, and the molding auxiliary line 420 can be rolled up by rotating the winding assembly 320. The winding assembly 320 is generally a rotating shaft, and one end of the molding auxiliary line 420 is fixed on the rotating shaft. Optionally, a knob is provided at the connecting rotating shaft to facilitate the doctor to rotate the winding assembly 320.

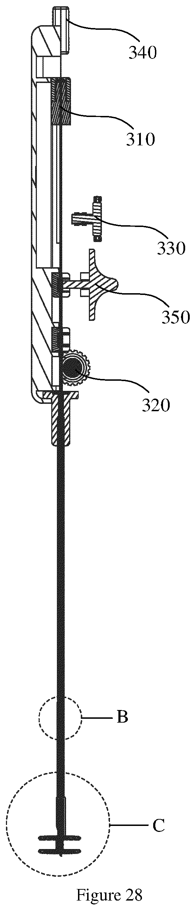

In some embodiments, please refer to Figures 28-29, and a recovery line 500 is also included, which is wound around the end of the second section 130 away from the third section 110 and passes through the first section 120 and the third section 110. The two ends of the recovery line 500 are fixed on the operating mechanism 30. Of course, the recovery line 500 also has a guiding function, because the recovery line 500 is inserted into the recovery tube 210, and the recovery line 500 can guide the movement of the recovery tube 210, thereby ensuring that the occluder 10 can be restored from a double disc shape to a tubular shape.

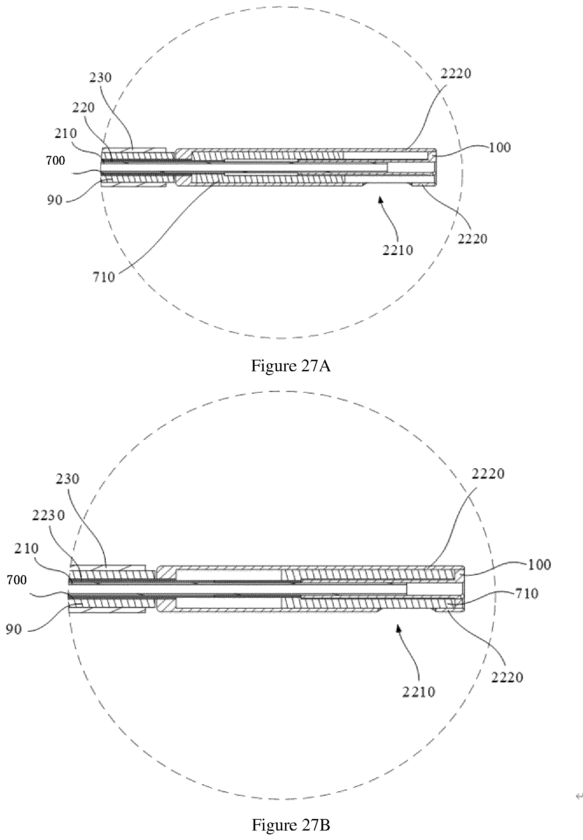

In some embodiments, please refer to Figure 26, Figure 27A and Figure 27B, the connecting mechanism 20 also includes a line-restraining tube 230, and the connecting tube 220 is sleeved in the line-restraining tube 230. Generally, the molding auxiliary line 420 is outside the connecting tube 220, and the molding auxiliary line 420 can be bundled in the line-restraining tube 230 to avoid possible contamination of the molding auxiliary line 420. In some embodiments, the line-restraining tube 230 also includes a second through hole 2310 to facilitate the insertion of the molding auxiliary line 420. Since the molding auxiliary line 420 is arranged between the line-restraining tube 230 and the connecting tube 220, a second through hole 2310 is provided on the line-restraining tube 230 to facilitate the insertion of the molding auxiliary line 420.

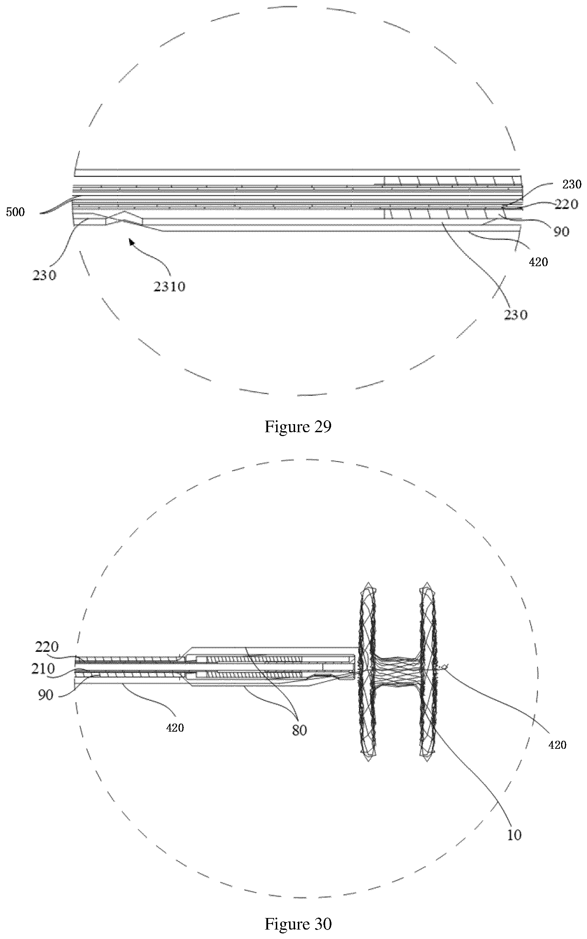

In some embodiments, please refer to Figure 28 and Figure 30, and a withdrawal line 80 is also included, which is wound around the end of the first section 120 away from the third section 110, and the two ends of the withdrawal line 80 are fixed on the connecting tube 220. The function of the withdrawal line 80 is to facilitate the dragging of the occluder 10, and one end of the withdrawal line 80 is bound to the end of the occluder 10 close to the connecting tube 220. When the occluder 10 needs to be recovered, the occluder 10 can be released by repositioning it after the occluder 10 becomes tubular.

In some embodiments, heat shrink tube 90 is provided at both ends of the withdrawal line 80 to fix the withdrawal line 80. The withdrawal line 80 needs to be fixed, so heat shrink tube 90 is provided at both ends of the withdrawal line 80. By heating the heat shrink tube 90, the two ends of the withdrawal line 80 are fixed to the outside of the connecting tube 220.

In some embodiments, please refer to Figure 25, Figure 26, Figure 27A and Figure 27B, the connecting mechanism 20 also includes a cutting line tube 700, a first through hole 2210 is provided on the connecting tube 220. The cutting line tube 700 is sleeved between the recovery tube 210 and the connecting tube 220 and can slide, the withdrawal line 80 passes through the first through hole 2210. When the cutting line tube 700 and the connecting tube 220 move relative to each other, the port of the cutting line tube 700 can cut off the withdrawal line 80 at the first through hole 2210, and the operating mechanism 30 has a cutting-line switch 350 for dragging the cutting line tube 700 to move.

After the occluder 10 is correctly installed in the patient's part, the occluder 10 and the connecting mechanism 20 need to be separated at this time. The occluder 10 and the connecting mechanism 20 are generally screwed, and the occluder 10 and the connecting mechanism 20 are separated by rotating the connecting mechanism 20. Although the withdrawal line 80 is made of a degradable material, it is best not to leave too much in the human body. At this time, the moving cutting-line switch 350 drives the cutting line tube 700 to move, so that the cutting line tube 700 and the first through hole 2210 of the connecting tube 220 form a shearing force on the withdrawal line 80. Finally, the withdrawal line 80 broken into two parts is pulled out at the operating mechanism 30.

In the present invention, the occluder 10 and the connecting mechanism 20 can be connected by the withdrawal line 80. The occluder 10 and the connecting mechanism 20 can be disconnected by cutting off the withdrawal line 80.

In some embodiments, the connecting tube 220 is provided with a first through hole 2210, and the molding auxiliary line 420 passes through the first through hole 2210. When the cutting line tube 700 and the connecting tube 220 move relative to each other, the port of the cutting line tube 700 can cut off the molding auxiliary line 420 at the first through hole 2210. Similarly, the molding auxiliary line 420 can also be cut off, but part of the molding auxiliary line 420 must remain in the human body. The molding auxiliary line 420 can also be degraded.

In some embodiments, the operating mechanism 30 also includes a locking switch 330. Pressing the locking switch 330 can lock the gripping member 310, and the gripping member 310 can be a grip. The function of the locking switch 330 is to lock the gripping member 310 to prevent the doctor from misoperating and causing the gripping member 310 to move and change the shape of the occluder 10. The locking switch 330 can be a knob type or a button type. As long as it can limit the movement of the gripping member 310, it belongs to the protection scope of the present invention.

In some embodiments, the operating mechanism 30 also includes two fixing blocks 340, and the two fixing blocks 340 fix one end of the recovery line 500 respectively. The fixing block 340 is generally detachably connected to the main body of the operating mechanism 30, and the recovery line 500 is moved out by pulling the fixing block 340. Generally, the recovery line 500 is cut at the operating mechanism 30, and finally all the recovery lines 500 are pulled out.

In some embodiments, please refer to Figure 26, Figure 27A and Figure 27B, the connecting tube 220 includes a flexible section 2230 and a rigid section 2220. The first through hole 2210 is arranged on the rigid section 2220, and the cutting line tube 700 is provided with a second cutting member 710 at one end close to the occluder 10. Since the cutting-line needs to be rigid, the connecting tube 220 is composed of a flexible section 2230 and a rigid section. The cutting line tube 700 is provided with a second cutting member 710 at one end close to the occluder 10, and the second cutting member 710 cooperates with the rigid section to cut line better. The second cutting member 710 and the rigid section can be nickel-titanium alloy or stainless steel.

It should be noted that the diameters of the flexible section 2230 and the rigid section 2220 may be different. Generally, the diameter of the rigid section 2220 is larger. In some embodiments, the flexible section 2230 and the rigid section 2220 are fixedly connected, and the second cutting member 710 and the cutting line tube 700 are fixedly connected. In this embodiment, the flexible section 2230 is fixedly connected to the rigid section, and the second cutting member 710 is fixedly connected to the cutting line tube 700. The second cutting member 710 and the rigid section 2220 can be installed later to reduce the difficulty of the integrated molding assembly of the connecting mechanism 20.

In some embodiments, a sheath tube 100 is also included, and the outer peripheral surface of the sheath tube 100 is provided with a second cutting member 710. The existence of the sheath tube 100 is to provide a guide for the recovery tube 210. Generally, it is assembled separately with the second cutting member 710 and the rigid section 2220 during the production process.

Note that the above are only preferred embodiments of the present invention and the technical principles used. Those skilled in the art will understand that the present invention is not limited to the specific embodiments here, and that various obvious changes, readjustments and replacements can be made by those skilled in the art without departing from the scope of protection of the present invention. Therefore, although the present invention is described in more detail through the above embodiments, the present invention is not limited to the above embodiments, and may also include more other equivalent embodiments without departing from the scope of the present invention. The scope of the present invention is determined by the scope of the attached claims.

The above is only a preferred embodiment of the present invention and is not intended to limit the present invention. Any modifications, equivalent replacements, improvements, etc. made within the spirit and principles of the present invention shall be included in the protection scope of the present invention.

### INDUSTRIAL APPLICABILITY

The present invention reduces the inner diameter of the channel formed at the end of the second occluding portion by providing a lacing member and a limiting member to improve the locking success rate of the occluder during surgery. In addition, the recyclable occluder system provided by the present invention can solve the problem that the occluder cannot be retracted after the umbrella body is formed.

## Claims

1. An occluder, **characterized in that** the occluder comprises a first occluding portion and a second occluding portion, and the first occluding portion and the second occluding portion are connected by a connecting portion; and
the first occluding portion and the second occluding portion are both composed of a mesh structure, the second occluding portion is provided with a lacing member, the lacing member is arranged in a surrounding manner and passes through meshes at an end of the second occluding portion in sequence, and a limiting member is arranged on the lacing member, the limiting member comprises a plurality of limiting segments, and the plurality of limiting segments are connected and enclosed on the lacing member to form a closed pattern.

2. The occluder according to claim 1, wherein the first occluding portion is further provided with a lacing member, the lacing member is arranged in a surrounding manner and passes through meshes at an end of the first occluding portion in sequence; and the closed pattern formed by connection and enclosure of the plurality of limiting segments on the lacing member comprises any one of a star shape, a tic-tac-toe shape or a triangle.

3. The occluder according to claim 1, wherein the limiting member comprises an enclosure and two or more extensions, the enclosure is connected end to end to form a closed pattern, one end of the extension is arranged on the enclosure, and the other end extends away from the enclosure and is arranged on the lacing member, and the two or more extensions are arranged at intervals along a circumference of the enclosure to fix the enclosure on the lacing member.

4. An occluder manufacturing device, **characterized in that** the occluder manufacturing device is configured to manufacture the occluder according to any one of claims 1 to 3, and the occluder manufacturing device comprises a columnar body, wherein a first end of the columnar body is provided with a plurality of first positioning members (A1₁, A1₂...A1ₙ) and a plurality of second positioning members (A2₁, A2₂...A2ₙ), and the second positioning member at the first end of the columnar body is located between two adjacent first positioning members of the first end of the columnar body; and a second end of the columnar body is further provided with a plurality of first positioning members (B1₁, B1₂...B1ₙ) and a plurality of second positioning members (B2₁, B2₂...B2ₙ); and the second positioning member at the second end of the columnar body is located between two adjacent first positioning members of the second end of the columnar body; and
the occluder manufacturing device is suitable for an occluder weaving method for manufacturing the occluder according to any one of claims 1 to 3, and the occluder weaving method comprises: fixing a single wire on A1₁, and passing the single wire through all the first positioning members and the second positioning members in an order of A1₁, B1₁, A2₁, B2₁...A1ₙ, B1ₙ, A2ₙ, B2ₙ to form a weaved body, wherein the single wire located between the first end of the columnar body and the second end of the columnar body is wound along an outer circumference of the columnar body, wherein, during a weaving process, the single wire is interwoven and passed through a weaved paths up and down to form a mesh structure; fixing a middle portion of the weaved body, and squeezing two ends of the weaved body toward the middle portion of the weaved body to form an occluder having the first occluding portion and the second occluding portion.

5. The occluder manufacturing device according to claim 4, wherein a detachable connection structure is provided between at least one of the first positioning member and the second positioning member and the columnar body to adjust a distance between two adjacent first positioning members and a distance between two adjacent second positioning members;
and/or, a sliding connection structure is provided between at least one of the first positioning member and the second positioning member and the columnar body to adjust a distance between adjacent first positioning member and second positioning member;
and/or, the distance between two adjacent first positioning members is between 1mm and 10mm;
and/or, the distance between two adjacent second positioning members is between 1mm and 10mm;
and/or, the distance between the adjacent first positioning member and second positioning member is between 2mm and 10mm;
and/or, a height of the first positioning member is between 1mm and 10mm;
and/or, a height of the second positioning member is between 1mm and 10mm.

6. An occluder system, **characterized in that** the occluder system comprises: the occluder according to any one of claims 1 to 3, a conveying and cutting device; and a first auxiliary line, wherein one end of the first auxiliary line is arranged on an end surface of the first occluding portion of the occluder away from the second occluding portion of the occluder, the other end of the first auxiliary line passes through the second occluding portion through the limiting member of the second occluding portion and is connected to the conveying and cutting device, and the first auxiliary line is provided with two or more clamping parts, the clamping parts are configured to pass through the second occluding portion through the limiting member under an action of external force, and clamp with the limiting member when the external force disappears.

7. The occluder system according to claim 6, wherein the occluder system further comprises:
a second auxiliary line, wherein the second auxiliary line is connected to the second occluding portion and is configured to drive the occluder to move under the action of external force; and
the conveying and cutting device is connected in advance with the occluder through the first auxiliary line and the second auxiliary line.

8. The occluder system according to claim 7, wherein the conveying and cutting device comprises a first tube body, a second tube body, a third tube body, a conveying member, a first cutting member and a handle, one end of the conveying member is fixed on the handle, and the other end of the conveying member passes through the second tube body and the third tube body in sequence and then is connected to the first tube body; the first auxiliary line extends into an end of the first tube body, exits through the first tube body from a side wall of the first tube body, and then is located between the conveying member and the second tube body; the second auxiliary line extends into the end of the first tube body, exits through the first tube body from the side wall of the first tube body, and then is located between the conveying member and the third tube body; one end of the first cutting member is arranged on the handle, and the other end of the first cutting member passes through the second tube body and the third tube body in sequence and then is located in the first tube body, and the first cutting member is configured to move relative to the first tube body under the drive of the handle to cut the first auxiliary line and the second auxiliary line.

9. The occluder system according to claim 8, wherein the cutting member comprises a driving member and a cutting head, the cutting head is movably arranged in the first tube body, one end of the driving member is movably arranged on the handle, and the other end of the driving member passes through the second tube body and the third tube body in sequence and then extends into the first tube body to connect with the cutting head, and the driving member is configured to move relative to the handle under the drive of the handle, thereby driving the cutting head to move relative to the first tube body.

10. The occluder system according to any one of claims 6 to 9, wherein a first end of the clamping part is closer to the first occluding portion than a second end of the clamping part, and a distance between an outer wall of the first end of the clamping part and the first auxiliary line is greater than a distance between an outer wall of the second end of the clamping part and the first auxiliary line.

11. The occluder system according to any one of claims 6 to 10, wherein an angle between an outer wall of the clamping part and the first auxiliary line is between 10° and 80°; and/or, the first end of the clamping part is recessed toward the second end of the clamping part to form a recessed portion, and an angle between a side wall of the recessed portion and the first auxiliary line is between 0° and 75°; and/or, the first end of the clamping part is recessed toward the second end of the clamping part to form a recessed portion, and the recessed portion makes the first end of the clamping part sharp-pointed; and/or, the clamping part is in a shape of a fishbone barb.

12. The occluder system according to claim 8 or 9, wherein an end cap is provided at an end of the first tube body, and a channel is provided on the end cap, and the first auxiliary line and the second auxiliary line are both extended into the first tube body through the channel and exit through the first tube body through a side wall of the first tube body.

13. The occluder system according to claim 8 or 9, wherein at least three identification members are arranged on the second tube body, and the at least three identification members comprise a first identification member, a second identification member and a third identification member arranged at intervals; and
when the occluder system is in use, and the first identification member moves to a set position, the first occluding portion and the second occluding portion are both in a retracted state; when the second identification member moves to a set position, the first occluding portion is in a released state, and the second occluding portion is in a retracted state; and when the third identification member moves to a set position, the first occluding portion and the second occluding portion are both in a released state.

14. A retrievable occluder system, **characterized in that** the retrievable occluder system comprises: an occluder having a tubular third section and a first section and a second section capable of switching between tubular and disc-shaped forms, wherein two ends of the third section are respectively connected to the first section and the second section; and
a connecting mechanism, wherein a recovery tube and a connecting tube are sequentially sleeved from inside to outside, wherein the recovery tube can move along a length direction of the connecting mechanism; an operating mechanism, having a gripping member capable of dragging the recovery tube to move; a molding auxiliary line, wherein one end of the molding auxiliary line is fixed to an end of the second section away from the third section, and the other end of the molding auxiliary line is fixed to the operating mechanism; and one end of the connecting tube is fixed to the operating mechanism, and the other end of the connecting tube is detachably connected to the occluder.

15. The retrievable occluder system according to claim 14, wherein the molding auxiliary line is provided with a node at one end close to the occluder, wherein the node can pass through and be stuck at an outer closed end of the first section; and/or the node is any one of a knot, a fishbone line or a spring fastener.

16. The retrievable occluder system according to claim 14 or 15, wherein the operating mechanism further comprises a winding assembly, the molding auxiliary line is fixed on the winding assembly, and the molding auxiliary line can be wound by rotating the winding assembly.

17. The retrievable occluder system according to claim 14 or 15, wherein the retrievable occluder system further comprises a recovery line, the recovery line is wound around an end of the second section away from the third section and passes through the first section and the third section, and two ends of the recovery line are fixed on the operating mechanism; the connecting mechanism further comprises a line-restraining tube, and the connecting tube is sleeved in the line-restraining tube;
and/or, the line-restraining tube further comprises a second through hole to facilitate insertion of the molding auxiliary line.

18. The retrievable occluder system according to any one of claims 14 to 17, wherein the retrievable occluder system further comprises a withdrawal line, the withdrawal line is wound around an end of the first section away from the third section, and two ends of the withdrawal line are fixed on the connecting tube; and heat shrink tube is provided at both ends of the withdrawal line to fix the withdrawal line.

19. The retrievable occluder system according to claim 18, wherein the connecting mechanism further comprises a cutting line tube, the connecting tube is provided with a first through hole, the cutting line tube is sleeved between the recovery tube and the connecting tube and can slide, the withdrawal line passes through the first through hole, when the cutting line tube and the connecting tube move relative to each other, a port of the cutting line tube can cut off the withdrawal line at the first through hole, and the operating mechanism has a cutting-line switch for dragging the cutting line tube to move; and
the connecting tube is provided with a first through hole, the molding auxiliary line passes through the first through hole, and when the cutting line tube and the connecting tube move relative to each other, a port of the cutting line tube can cut off the molding auxiliary line at the first through hole.

20. A retrievable occluder system, **characterized in that** the retrievable occluder system comprises: an occluder having a tubular third section and a first section and a second section capable of switching between tubular and disc-shaped forms, wherein two ends of the third section respectively connect the first section and the second section; a molding auxiliary line, wherein one end of the molding auxiliary line is fixed to an end of the second section away from the third section, and the other end of the molding auxiliary line is fixed to an operating mechanism, and the molding auxiliary line is configured to tighten the first section and the second section; and a withdrawal line, wherein the withdrawal line is wound around an end of the first section away from the third section, two ends of the withdrawal line are fixed to a connecting tube, and the withdrawal line is configured to withdraw the occluder under an action of an external force.
